(19)

**Europäisches Patentamt**
**European Patent Office**
**Office européen des brevets**

(11) **EP 1 668 764 B1**

(12) **EUROPEAN PATENT SPECIFICATION**

(45) Date of publication and mention
of the grant of the patent:
**27.08.2008 Bulletin 2008/35**

(51) Int Cl.:
**H02K 7/09** (2006.01)   **H02K 7/14** (2006.01)
**H02K 21/24** (2006.01)

(21) Application number: **04794058.0**

(86) International application number:
**PCT/US2004/032560**

(22) Date of filing: **01.10.2004**

(87) International publication number:
**WO 2005/034312 (14.04.2005 Gazette 2005/15)**

(54) **ROTARY PUMP WITH ELECTROMAGNETIC LCR BEARING**

DREHPUMPE MIT ELEKTROMAGNETISCHEM LCR-LAGER

POMPE ROTATIVE A SUPPORT LCR ELECTROMAGNETIQUE

(84) Designated Contracting States:
**AT BE BG CH CY CZ DE DK EE ES FI FR GB GR HU IE IT LI LU MC NL PL PT RO SE SI SK TR**

(30) Priority: **03.10.2003 US 508618 P**

(43) Date of publication of application:
**14.06.2006 Bulletin 2006/24**

(73) Proprietors:
• **FOSTER-MILLER, INC.**
  **Waltham,**
  **MA 02154 (US)**
• **THE CLEVELAND CLINIC FOUNDATION**
  **Cleveland, OH 44195 (US)**

(72) Inventors:
• **CHEN, Hsiang, Ming**
  **Latham, NY 12110 (US)**
• **SMITH, William, A.**
  **Lyndhurst, OH 44124 (US)**
• **VITALE, Nicholas, G.**
  **Albany, NY 12203 (US)**
• **CHAPMAN, Peter, A., Jr.**
  **East Schodack, NY 12063 (US)**
• **DONAHUE, Arthur, C.**
  **East Greenbush, NY 12061 (US)**
• **MEEKER, David, C.**
  **Natick, MA 01760 (US)**
• **PRISCO, Charles, J.**
  **Saratoga Springs, NY 12866 (US)**

(74) Representative: **Naismith, Robert Stewart et al**
**Marks & Clerk**
**19 Royal Exchange Square**
**Glasgow**
**G1 3AE (GB)**

(56) References cited:
EP-A- 0 901 797        EP-A- 1 113 177
US-A- 5 078 741        US-A- 6 126 638

• SHEN J X ET AL: "A novel compact PMSM with magnetic bearing for artificial heart application" INDUSTRY APPLICATIONS CONFERENCE, 1999. THIRTY-FOURTH IAS ANNUAL MEETING. CONFERENCE RECORD OF THE 1999 IEEE PHOENIX, AZ, USA 3-7 OCT. 1999, PISCATAWAY, NJ, USA,IEEE, US, vol. 2, 3 October 1999 (1999-10-03), pages 1201-1207, XP010355293 ISBN: 0-7803-5589-X
• CHEN H M ET AL: "HIGH EFFICIENCY MAGNETIC BEARING FOR A ROTARY BLOOD PUMP" ASAIO JOURNAL, J.B.LIPPINCOTT CO.,HAGERSTOWN, MD, US, vol. 44, no. 5, September 1998 (1998-09), pages M728-M732, XP000802392 ISSN: 1058-2916
• DATABASE WPI Section PQ, Week 200349 Derwent Publications Ltd., London, GB; Class P34, AN 2003-514741 XP002319970 & CN 1 414 687 A (UNIV SHENYANG POLYTECHNIC) 30 April 2003 (2003-04-30)

EP 1 668 764 B1

**Description**

**Related Application**

This application claims the benefit of U.S. Provisional Application No. 60/508,618, which was filed on October 2, 2003.

**Technical Field**

The present invention relates to a rotary pump with an electromagnetic LCR bearing for supporting the pump rotor.

**Background of the Invention**

Heart disease is the leading cause of death and disability in the United States. Some approaches to the treatment of heart disease employ the use of pumping systems that are used to assist the heart in its pumping function, bypass the heart, or replace the heart. Such pumping systems may include pumps that are implantable or pumps that remain external to the patient. One example of a pumping system is a post-cardiotomy assist system. Another example of a pumping system is a bridge-to-transplant system for assisting or replacing a patient's heart while awaiting a transplant. A further example of a pumping system is a bridge-to-recovery system, such as a ventricular assist device (VAD), that assists the patient's heart in order to promote myocardial recovery, either spontaneously, with drugs, or with gene therapy.
Document US 5,078,741 discloses a prior art pump with a magnetically supported impeller.

**Summary of the Invention**

The present invention relates to a pump including a housing having a fluid inlet and a fluid outlet. A rotor is disposed within the housing and is rotatable about an axis to move fluid from the fluid inlet to the fluid outlet. A magnetic axial bearing supports the rotor. The axial bearing includes an axial bearing target disposed on the rotor and an axial bearing stator disposed on the housing. The axial bearing stator includes multiple stator poles, each of the stator poles including a first coil portion wound in a first direction and a second coil portion wound in a second direction opposite the first direction.
The present invention also relates to a pump including a housing having a fluid inlet and a fluid outlet. A rotor is disposed within the housing and is rotatable about an axis to move fluid from the fluid inlet to the fluid outlet. A magnetic first radial bearing exerts a force on the rotor in a first direction along the axis and a magnetic second radial bearing exerts a force on the rotor in a second direction along the axis opposite the first direction. The first and second radial bearings are adjustable to allow for independently adjusting the net axial force exerted on the rotor by the radial bearings and the radial stiffness of the radial bearings.
The present invention also relates to a pump including a housing having a fluid inlet and a fluid outlet. A rotor is disposed within the housing and is rotatable about an axis to move fluid from the inlet to the outlet. The rotor includes permanent magnets arranged on a first side of the rotor and a magnetically conductive disk arranged on a second side of the rotor opposite the first side of the rotor. A motor stator is arranged on the housing to interact with the permanent magnets on the rotor. At least one electromagnet is arranged on the housing to interact with the magnetically conductive disk on the rotor. At least one ring magnet is arranged on at least one of the first and second sides of the rotor. At least one ring magnet is arranged on the housing to magnetically interact with the at least one ring magnet on the rotor.
The present invention also relates to a pump including a housing having a fluid inlet and a fluid outlet. A rotor is disposed within the housing and is rotatable about an axis to move fluid from the inlet to the outlet. A motor is arranged to cause rotation of the rotor. At least one electromagnet is arranged to interact magnetically with material in the rotor. The electromagnet includes a stator formed from a spiral wound lamination material.
The present invention also relates to a pump including a housing having a fluid inlet and a fluid outlet. A rotor is disposed within the housing and is rotatable about an axis to move fluid from the inlet to the outlet. A motor is arranged to cause rotation of the rotor. At least one electromagnet is arranged to interact magnetically with material in the rotor. The electromagnet includes an even number of poles, adjacent poles being wound in opposite directions.
The present invention also relates to a pump including a housing having a fluid inlet and a fluid outlet. A rotor is disposed within the housing and is rotatable about an axis to move fluid from the inlet to the outlet. A motor is arranged to cause rotation of the rotor. At least one electromagnet is arranged to interact magnetically with material in the rotor. The material includes a disk of spiral wound magnetic alloy material.
The present invention also relates to a method for magnetically supporting a pump rotor in a housing for rotation about an axis. The method includes the step of providing an axial bearing target on the rotor. The method also includes the step of providing an axial bearing stator on the housing, the axial bearing stator including multiple stator poles, each including a first coil portion and a second coil portion. The method also includes the steps of winding the first coil portion in a first direction and winding the second coil portion in a second direction opposite the first direction.

The present invention further relates to a method for magnetically supporting a pump rotor in a housing for rotation about an axis. The method includes the step of providing a magnetic first radial bearing for exerting a force on the rotor in a first direction along the axis. The method also includes the step of providing a magnetic second radial bearing for exerting a force on the rotor in a second direction along the axis opposite the first direction. The method further includes the step of adjusting the axial positions of the first and second radial bearings to independently adjust the net axial force exerted on the rotor by the radial bearings and the radial stiffness of the radial bearings.

## Brief Description of the Drawings

The foregoing and other features of the present invention will become apparent to those skilled in the art to which the present invention relates upon reading the following description with reference to the accompanying drawings, in which:

Fig. 1 is a perspective view of a pump apparatus according to the present invention;
Fig. 2 is an exploded view of the apparatus of Fig. 1;
Figs. 3A and 3B are side views illustrating a portion of the apparatus of Figs. 1 and 2;
Figs. 4A and 4B are side views illustrating alternative configurations of a portion of the apparatus of Figs. 1 and 2;
Figs. 5A-5C are schematic illustrations depicting the operation of a portion of the apparatus of Figs. 1 and 2;
Fig. 6A is a schematic illustration depicting the operation of a portion of the apparatus of Figs. 1 and 2; and
Fig. 6B is a graph illustrating certain operating parameters of the apparatus of Figs. 1 and 2.

## Description of Embodiments

The present invention relates to a rotary pump that includes magnetic bearings for supporting the pump rotor by magnetically levitating the rotor. In one embodiment, the pump may comprise a blood pump for incorporation in a system for pumping blood in a patient. For example, the pump may be a cardiac assist pump or a cardiac replacement pump. The present invention, however, is not necessarily limited to blood pumps and could have alternative implementations or uses in which the pump is used to pump alternative fluids.

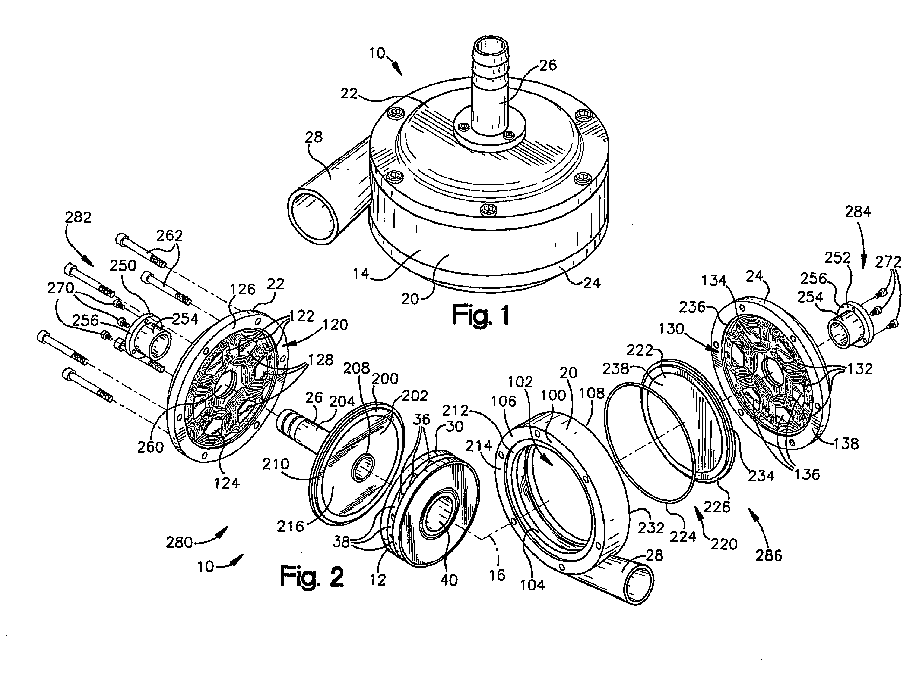

Figs. 1 and 2 illustrate an example configuration of an apparatus in the form of a pump 10 for pumping fluids. The pump 10 may, for example, be a blood pump. In the embodiment of the invention illustrated in Figs. 1 and 2, the pump 10 is a rotary pump in which a rotor assembly or rotor 12 is supported in a housing 14 for rotation about an axis 16. The housing 14 includes a central volute housing part 20, a motor stator housing part 22, and an inherently controlled bearing (ICB) stator housing part 24. The motor stator housing 22 and ICB housing 24 are connectable to the volute housing 20 on opposite sides of the volute housing, thus forming the assembled condition of the housing 14 shown in Fig. 1. The pump 10 includes an inlet 26, associated with the motor stator housing 22, through which fluid is directed into the pump. The pump 10 also includes an outlet 28, associated with the volute housing 20, through which fluid is discharged from the pump.

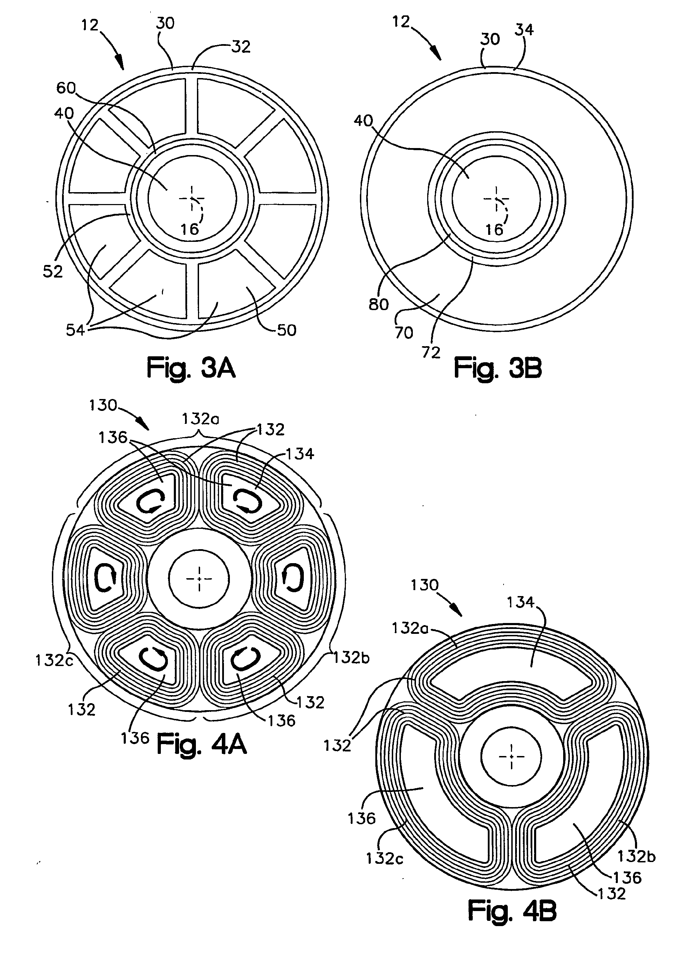

Referring to Figs. 2, 3A, and 3B, the rotor 12 includes an impeller structure 30 that includes a generally disk shaped motor side end wall 32 and a generally disk shaped ICB side end wall 34. The end walls 32 and 34 are spaced from each other, parallel to each other, and centered on the axis 16. The impeller structure 30 also includes a plurality of impeller vanes 36 that extend between the end walls 32 and 34 and help define a plurality of impeller passages 38. A central axially extending inlet passage 40 extends through the rotor 12 is in fluid communication with the impeller passages 38.

Referring to Figs. 2 and 3A, the motor side end wall 32 supports a motor permanent magnet (PM) ring 50 and a motor side PM radial bearing ring 60 of the rotor 12. The motor side PM radial bearing ring 60 is positioned radially inward of the motor PM ring 50. An annular insulating portion 52 may help isolate the motor PM ring 50 and the motor side PM radial bearing ring 60. The motor PM ring 50 and motor side PM radial bearing ring 60 are fixed to or embedded in the motor side end wall 32 of the rotor 12. The motor PM ring 50 includes a plurality of PM motor magnets 54 arranged in an annular fashion about the motor side end wall 32. In the embodiment illustrated in Fig. 2, the motor PM ring 50 includes eight (8) motor magnets 54 equal in size and arranged spaced evenly in an annular fashion about the motor PM ring 50. The motor PM ring 50 could, however, have an alternative configuration, such as including a different number of PM motor magnets 54.

The motor side PM radial bearing ring 60 may be made of a permanent magnet material with high coercivity such as neodymium boron iron. The motor side PM radial bearing ring 60 has an annular single magnet construction. The motor side PM radial bearing ring 60 could, however, have an alternative construction. For example, the motor side PM radial bearing ring 60 may include multiple magnet segments and may include flux carrying material, such as iron, to aid in creating a desired magnetic flux path.

Referring to Figs. 2 and 3B, the bearing side end wall 34 supports a ring-shaped axial bearing target 70 and a bearing side PM radial bearing ring 80 of the rotor 12. The bearing side PM radial bearing ring 80 is positioned radially inward of the axial bearing target 70. An annular insulating portion 72 of the bearing side end wall 34 may help isolate the axial bearing target 70 and the bearing side PM radial bearing ring 80. The axial bearing target 70 and bearing side PM radial bearing ring 80 are fixed to or embedded in the bearing side end wall 34 of the rotor 12. The axial bearing target 70 has an annular configuration and is constructed of ferrite, powder iron, or laminated silicon steel. For example, the axial bearing target 70 could be formed from a thin flat strip or strips of material that are wound to form the axial bearing target. The axial bearing target 70 could, however, have an alternative configuration, an alternative material construction, or both.

The bearing side PM radial bearing ring 80 may be made of a permanent magnet material with high coercivity such as neodymium boron iron. The bearing side PM radial bearing ring 80 has an annular single magnet construction. The bearing side PM radial bearing ring 80 could, however, have an alternative construction. For example, the bearing side PM radial bearing ring 80 may include multiple magnet segments and may include flux carrying material, such as iron, to aid in creating a desired magnetic flux path.

The volute housing part 20 (Fig. 2) has a generally cylindrical main portion 100 that helps define a volute pump chamber 102. The main portion 100 has a first end forming a motor side 106 of the volute housing 20 and an opposite second end forming a stator side 108 of the volute housing. The pump chamber 102 is sized to receive the rotor 12 and form a predetermined clearance with the rotor. An annular pumping channel 104 is formed in the main portion 100 an is in fluid communication with the pump chamber 102 and the impeller passages 38 when the pump 10 is assembled. The outlet 28 extends generally tangentially from the main portion 100 of the volute housing part 20 and is in fluid communication with the pumping channel 104.

The motor stator housing 22 is adapted to support a motor stator assembly 120 of the pump 10. In the illustrated embodiment, the motor stator assembly 120 includes six (6) motor stator coils 122 arranged in an annular configuration. Each motor stator coil 122 is made of copper wire that is wound in layers around posts 128 formed on a core 124. The core 124 may be formed of a ferromagnetic material, such as a powdered iron material or a silicon steel material, and thereby serve as a flux return path. This may help improve the torque per unit coil current ratio of the pump 10. Alternative materials could be used to form the motor stator coils 122, the core 124, or both.

The motor stator assembly 120 is connected to the motor stator housing 22 by means (not shown) such as an adhesive. Alternative means may be used to secure the motor stator assembly 120 to the motor stator housing 22. The motor stator assembly 120, particularly the motor stator coils 122, may also be sealed or covered with a material, such as a film, that is coextensive with an annular inner surface 126 of the motor stator housing 22. The assemblage of the motor stator housing 22 and motor stator assembly 120 may thus have a generally flat smooth surface exposed to the pump chamber 102.

The bearing stator housing 24 is adapted to support an ICB bearing stator assembly 130 of the pump 10. Alternative embodiments for the bearing stator assembly 130 are illustrated in Figs. 4A and 4B, respectively. In the embodiment illustrated in Figs. 2 and 4A, the bearing stator assembly 130 includes six (6) bearing stator coils 132 arranged in an annular configuration. In the embodiment illustrated in Fig 4B, the bearing stator assembly 130 includes three (3) bearing stator coils 132 arranged in an annular configuration. In the embodiments of Figs. 4A and 4B, each bearing stator coil 132 is made of copper wire that is wound in layers around stator posts 136 on an axial bearing stator core 134. The axial bearing stator core 134 may be constructed of a ferromagnetic material and thus may serve as a flux return path. Alternative materials could be used to form the bearing stator coils 132, the axial bearing stator core 134, or both.

Referring to Fig. 2, the bearing stator assembly 130 is connected to the bearing stator housing 24 by means (not shown) such as an adhesive. Alternative means may be used to secure the bearing stator assembly 130 to the bearing stator housing 24. The bearing stator assembly 130, particularly the bearing stator coils 132, may also be sealed or covered with a material, such as a film, that is coextensive with an annular inner surface 138 of the bearing stator housing 24. The assemblage of the bearing stator housing 24 and bearing stator assembly 130 may thus have a generally flat smooth surface exposed to the pump chamber 102.

Referring to Fig. 4A, the six bearing stator coils 132 of the bearing stator assembly 130 are configured for three phase excitation. In this configuration, adjacent pairs of the bearing stator coils 132 are wound with the same wire and excited with the same voltage phase. In this configuration, three pairs of bearing stator coils form three bearing coil poles 132a, 132b, and 132c. Each bearing stator pole 132a-c can be excited with a different phase of the three phase voltage. In this configuration, the individual bearing stator coils 132 of the bearing poles 132a-c may be wound in opposite directions. For example, for each pole 132a-c, one coil 132 may be wound in a clockwise direction and the other coil may be wound in a counterclockwise direction. This is indicated generally by the arrows in Fig. 4A.

In the embodiment illustrated in Fig. 4B, the three bearing stator coils 132 of the bearing stator assembly 130 form three bearing stator poles 132a, 132b, and 132c. Referring to Fig. 4B, the three bearing stator poles 132a-c of the bearing stator assembly 130 are configured for three phase excitation. Each bearing stator pole 132a-c can be excited with a different phase of the three phase voltage.

Referring to Fig. 2. the pump 10 also includes a motor seal assembly 200 that includes a generally disk shaped motor side seal 202 and a tubular port 204 that projects centrally from the motor side seal. The port 204 defines the inlet 26 of the pump 10. The motor side seal 202 includes an annular bead 210 that is adapted to be received in an annular shoulder or recess 212 in a motor side surface 214 of the volute housing part 20. A generally planar membrane portion 216 extends radially inward from the annular recess 212. As an example, the motor side seal 202, particularly the membrane portion 216, may be constructed of a sheet of polycarbonate connected to the bead 210 by an adhesive. The motor side seal 202 may have a mechanical stiffness derived from radial tensioning of the seal, back support from the motor stator 120, or both.

The port 204 projects perpendicularly from the membrane portion 216 and extends along the axis 16. The port 204 may be connected to the membrane portion 216 by an adhesive. The port 204 could project from the membrane at some other angle or could have an extent other than along the axis 16, such as a curved extent. As shown in Fig. 2, an annular projection 208 may project opposite the port 204. The projection 208 may help direct fluid flow into the inlet passage 40 of the rotor 12. The projection 208 may also serve as a small diameter or small surface area stop point for excessive axial travel of the rotor 12.

The pump 10 also includes a bearing seal assembly 220 that includes a generally disk shaped bearing side seal 222 and an O-ring 224. The bearing side seal 222 includes an annular groove 226 for receiving the O-ring 224. The volute housing part 20 may include a similarly or identically configured annular groove (not shown) for receiving the O-ring 224 that is recessed into a bearing side surface 232 of the volute housing part. The bearing side seal 222 also includes an annular bead 234 that is adapted to be received in an annular shoulder or recess 236 of the bearing stator housing 24. The bearing side seal 222 further includes a generally planar membrane portion 238 extends radially inward from the annular shoulder bead 234. As an example, the bearing side seal 222, particularly the membrane portion 238, may be constructed of a sheet of adhesive-backed polyester material mounted on the bead 234. The bearing side seal 222 may have a mechanical stiffness derived from radial tensioning of the seal, back support from the axial bearing stator 130, or both.

The pump 10 further includes a PM motor side radial bearing structure 250 connectable with the motor stator housing 22 and a PM bearing side radial bearing structure 252 connectable with the bearing housing 24. The motor side radial bearing structure 250 and the bearing side radial bearing structure 252 may be similarly or identically configured. In the illustrated embodiment, each of the radial bearing structures 250 and 252 includes a generally cylindrical PM bearing portion 254 and an annular flange portion 256 that extends radially outward from the PM bearing portion 254. The radial bearing structures 250 and 252 could have alternative configurations. The diameter and radial thickness of the PM bearing portions 254 may be similar or identical to that of the motor side radial bearing ring 60 and bearing side radial bearing ring 80. The PM bearing portions 254 may be made of a permanent magnet material with high coercivity such as neodymium boron iron. Also, the PM bearing portions may include multiple magnet segments and may include flux carrying material, such as iron, to aid in creating a desired magnetic flux path.

In an assembled condition of the pump 10, the motor stator assembly 120 is fixed to the motor stator housing 22 and the bearing stator assembly 130 is fixed to the bearing stator housing 24. The rotor 12 is positioned in the pump chamber 102 of the volute housing 20 such that the motor side end wall 32 of the impeller structure 30, the motor PM ring 50, and the motor side PM radial bearing ring 60 are positioned adjacent the motor side opening 106 of the volute housing. The ICB side end wall 34 of the impeller structure 30, the axial bearing target 70, and the bearing side PM radial bearing ring 80 are positioned adjacent the bearing side opening 108 of the volute housing 20.

The motor seal assembly 200 is positioned on the volute housing 20 with the annular bead 210 of the motor side seal 202 received in the annular recess 212 of the motor side surface 214. The membrane portion 216 of the motor side seal 202 extends radially inward across the motor side opening 106 of the volute housing 20. The tubular port 204 projects from the motor side seal 202 along the axis 16. The motor stator housing 22 is positioned on the motor side surface 214 such that the port 204 extends through a central opening 260 of the motor stator housing.

The O-ring 224 is placed in the annular groove 230 of the volute housing 20 and the bearing side seal 222 is placed on the volute housing such that the O-ring is received in the annular groove 226. The bearing stator housing 24 is then placed on the bearing side surface 232 of the volute housing 20 such that the annular bead 234 of the bearing side seal 222 is received in the recess 236 of the bearing stator housing. The membrane portion 238 of the bearing side seal 222 extends radially inward across the bearing side opening 108 of the volute housing 20.

Fastening means 262, such as machine screws, are used to secure the motor stator housing 22 and bearing stator housing 24 to the volute housing 20. This clamps the motor side seal 202 between the motor stator housing and the volute housing. This also clamps the bearing side seal 222 between the bearing stator housing and the volute housing. In the assembled condition of the pump 10, the bearing side seal assembly 200 isolates the motor stator assembly 120 from the pump chamber 102 and the bearing side seal assembly 220 isolates the bearing stator assembly 130 from the pump chamber.

In the illustrated embodiment, the motor side radial bearing 250 is connected to the motor stator housing 22 via means 270, such as machine screws, that extend through the flange portion 256 of the motor side radial bearing. Also, in the illustrated embodiment, the bearing side radial bearing 252 is connected to bearing housing 24 via means 272, such as machine screws, that extend through the flange portion 256 of the motor side radial bearing. Alternative means may be used to secure the radial bearings 250 and 252 to the housing 12.

The pump 10 illustrated in Figs. 1 and 2 is configured to have reusable parts and disposable parts. This may be a desirable configuration, for example, in the case of a non-implantable blood pump for short-term or medium-term use, such as an assist pump for use in a bridge-to-transplant or bridge-to-recovery scenario. This configuration is provided by the inclusion of the motor side seal assembly 200 and the bearing side seal assembly 220.

The motor side seal assembly 200 isolates the motor stator assembly 120 and the motor stator housing 22 from the pump chamber 102 and from any fluids (e.g., blood) in the pump chamber. Thus, during use of the pump 10, the motor stator assembly 120 and the motor stator housing 22 are not exposed to pumped fluids and thus may be reusable. Similarly, the bearing side seal assembly 220 isolates the bearing stator assembly 130 and the bearing stator housing 24 from the pump chamber 102 and from pumped fluids in the pump chamber. Thus, during use of the pump 10, the bearing stator assembly 130 and the bearing stator housing 24 are not exposed to pumped fluids and thus may be reusable. During use of the pump 10, the volute housing 20, rotor 12, motor side seal assembly 200, and bearing side seal assembly 220 are exposed to pumped fluids and thus may be disposable.

The pump 10 illustrated in Figs. 1 and 2 may also be configured as a disposable pump that has no reusable parts. This may be a desirable configuration, for example, in the case of an-implantable pump for medium-term or long-term use, such as a heart replacement pump. In this configuration, the motor side seal assembly 200 and the bearing side seal assembly 220 may not be required to isolate motor stator assembly 120 and motor stator housing 22 from the pump chamber 102. Similarly, in this configuration, the bearing side seal assembly 220 may not be required to isolate the bearing stator assembly 130 and the bearing stator housing 24 from the pump chamber 102. Thus, in the case where the pump 10 is disposable, the motor side seal assembly 200 and bearing side seal assembly 220 may not be necessary and can be omitted. In this instance, means, such as O-rings or gaskets, may be used to form a seal between the motor side housing 22 and the volute housing 20 and between the bearing side housing 24 and the volute housing. Also, in this instance, the inlet port 204 can be formed integrally with the motor side housing 22, can be fixed to the motor side housing by separate fastening means, or can be fixed to the motor side housing along with the motor side radial bearing 250.

The operation of the pump 10 is essentially the same, regardless of whether the pump is disposable or has reusable parts. The main difference between the configurations is a possible change in the effective magnetic gap between the magnets of the axial and radial bearings caused by the presence or absence of the motor seal assembly 200 and bearing seal assembly 220.

Referring to Fig. 2, a motor portion 280 of the pump 10 includes the motor stator assembly 120, the motor side end wall 32 of the rotor 12, and the motor PM ring 50. In the illustrated embodiment, the motor portion 280 is an eight pole, one sided axial gap permanent magnet brushless DC motor. The motor portion 280 has six motor stator coils 122 arranged for three phase excitation via, for example, a sinusoidal voltage source. It will be appreciated that alternative configurations for the motor portion 280 may also be implemented.

Also, referring to Fig. 2, a motor side radial bearing 282 of the pump 10 includes the motor side PM radial bearing ring 60 on the rotor 12 and the PM motor side radial bearing structure 250 on the motor stator housing 22. A bearing side radial bearing 284 of the pump 10 includes the bearing side PM radial bearing ring 80 on the rotor 12 and the PM bearing side radial bearing structure 252 on the bearing stator housing 24. An axial bearing 286 of the pump 10 includes the axial bearing target 70 on the rotor 12 and the ICB bearing stator assembly 130 on the bearing stator housing 24.

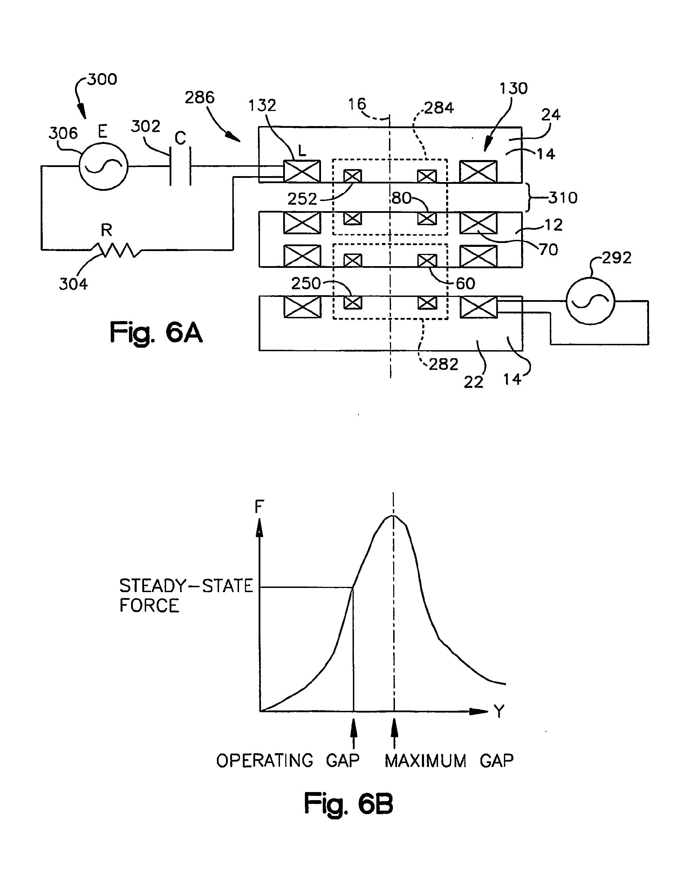

The pump is illustrated schematically in Fig. 6A. Referring to Fig. 6A, the magnets of the motor side and bearing side radial bearings 282 and 284 are arranged symmetrically with each other along the axis 16. The magnets of the radial bearings 282 and 284 are magnetized in the axial direction (e.g., along the axis 16) and arranged such that the magnets of each bearing are concentric with each other and attract each other. Thus, the motor side radial bearing ring 60 and the motor side radial bearing structure 250 are concentric with each other and attract each other. The bearing side radial bearing ring 80 and the bearing side radial bearing structure 252 are concentric with each other and attract each other. As a consequence, if the magnets of either radial bearing 282 and 284 become displaced radially from each other, the mutual attraction of the magnets in the axial direction will create a radial component force that tends to return the magnets to their concentric equilibrium position. This can be described as a "radial stiffness" of the radial bearings. The radial bearings 282 and 284 thus help maintain the radial position of the rotor 12 relative to the pump housing 14 and relative to the axis 16.

The axial bearing 286 helps control the axial linear and tilt positions of the rotor 12 relative to the pump housing 14. The position of the rotor 12 may be affected by a variety of factors, such as axial forces imposed on the rotor by the radial bearings 282 and 284, axial forces imposed by the motor portion 280 of the pump 10, fluid flow through the impeller structure 30, and gravity. The axial bearing 286, being configured for independent three phase excitation in either the three coil configuration (Fig. 4B) or six coil configuration (Fig. 4A), helps control the axial position of the rotor 12 along the axis and the tilt position of the rotor relative to the axis. To control the position of the rotor 12 along the axis 16, all three phases may be excited equally. To control the tilt position of the rotor 12 relative to the axis 16, the phases may be excited at different magnitudes.

The axial bearing 286 is configured such that excitation of the bearing stator coils 132 causes the coils and the axial bearing target 70 to attract each other. Excitation of the axial bearing 286 thus imposes an axial force on the rotor 12 that attracts the rotor toward the bearing stator assembly 130. The radial bearings 282 and 284 are configured to impose a net axial force on the rotor 12 in a direction opposite that imposed by the axial bearing 286. The position of the rotor 12 may thus be controlled via excitation of the axial bearing 286 to help maintain the rotor 12 in a desired levitated position.

Excitation of the axial bearing 286 is controlled via tuned LCR bearing circuits. There are three such LCR bearing circuits, one associated with each of the axial bearing stator poles 132a-c (see Figs. 4A and 4B). The LCR bearing circuits provide three phase excitation of the axial bearing stator 130. Each of the three LCR bearing circuits can be identical, with the exception of the difference of phase shift between the circuits. Each axial bearing stator pole 132a-c acts as an electromagnet when excited and thereby attracts the axial bearing target 70.

Fig. 6A depicts an example of a tuned LCR bearing circuit 300 illustrative of the circuit associated with each of the three axial bearing stator poles 132a-c. Each LCR bearing circuit 300 includes a coil 132 of an associated one of the bearing stator poles 132a-c connected in series with a capacitor 302, a resistor 304, and a voltage source 306. The resistor 304 could be in the form of a separate resistor component, the inherent resistance of the bearing stator poles, or both. The bearing stator poles 132a-c each have an inductance (L), the capacitor 302 has a capacitance (C), and the resistor 304 has a resistance (R). The voltage source 306 produces a sinusoidal voltage (E) that excites its associated bearing stator pole. The voltage (E) has a fixed frequency ($\omega$) that is slightly above a resonant frequency ($\omega_R$) of the circuit 300. The frequency ($\omega$) used in the LCR bearing circuit 300 for each coil 132 of the axial bearing 286 may be different. Also, for each LCR bearing circuit 300, different frequencies ($\omega$) may be used for start-up and steady state conditions of the pump 10.

As shown in Fig. 6A, the LCR circuit 300 is used to help suspend the rotor 12 via excitation of its associated bearing stator pole 132a-c of the axial bearing 286. According to the present invention, the LCR circuit provides self-sensing and self-positioning magnetic suspension of the rotor 12. The self-sensing and self-positioning magnetic suspension of the rotor 12 is illustrated in the force versus gap plot of Fig. 6B. Referring to Figs. 6A and 6B, if the rotor 12 moves away from the bearing stator 130, the magnetic gap 310 between the bearing stator and the axial bearing target 70 on the rotor increases and the inductance (L) decreases. As a result, the resonant frequency ($\omega_R$) of the circuit 300 increases, thus becoming closer to the excitation frequency ($\omega$) of the circuit. This causes an increase in the AC current generated by the voltage source 306, which increases the force with which the associated bearing stator pole 132a-c attracts the rotor 12. As a result, the axial force imposed on the rotor 12 pulls the rotor toward the associated bearing stator pole.

If the rotor 12 moves toward the bearing stator 130, the magnetic gap 310 decreases and the inductance (L) increases. As a result, the resonant frequency ($\omega_R$) of the circuit 300 decreases, thus increasing the difference between the resonant frequency and the excitation frequency ($\omega$). This causes a decrease in the AC current generated by the voltage source 306, which decreases the force with which the bearing stator pole 132a-c attracts the rotor 12. As a result, the axial force imposed on the rotor 12 by the bearing stator pole 132a-c is reduced and the axial force imposed on the rotor by the radial bearings 282 and 284 pulls the rotor away from the bearing stator 130. It will thus be appreciated that the LCR circuits 300 help maintain a fixed gap 310 between the bearing stator poles 132a-c and the axial bearing target 70 and helps maintain the position of the rotor 12.

It will also be appreciated that the excitation current may be used as an indication of the position of the rotor 12. This is because, as described above, the excitation current of the axial bearing 286 is proportional to the magnetic gap between the bearing stator 130 and the axial bearing target 70. Also, since the poles 132a-c of the axial bearing 286 control both the axial and tilt positions of the rotor 12, the excitation currents may be used to determine both the axial position of the rotor and the tilt position of the rotor.

The coils 122 of the motor stator 120 are excited via sinusoidal voltage source 292 shown schematically in Fig. 6A, which produces rotation of the rotor 12 and pumping action of the pump 10 in a known manner. The fluid to be pumped is drawn into the pump housing 14 (Fig. 2) through the inlet 26 and is directed through the impeller passages 38 into the pump chamber 102. The fluid fills the pump chamber 102 and is directed through the outlet 28 via the pumping channel 104.

The coils 122 of the motor stator 120 are excited with a signal having a frequency in the megahertz range via the sinusoidal voltage source 292. The coils 132 of the bearing stator 130 are excited with a signal having a frequency in the hundreds of hertz range via the sinusoidal voltage source 306. Those skilled in the art will appreciate that it may be desirable to superimpose the power for the bearing stator coils 132 on the power for the motor stator coils 122. The motor would thus require a single set of power leads. The power could be low pass filtered to the bearing stator coils 132 and either fed directly to or high pass filtered to the motor stator coils 122.

During operation of the pump 10, the fluid in the pump chamber 102 may provide damping for the rotor 12. More particularly, squeeze film damping may occur between the rotor 12 and the motor stator assembly 120 and between the rotor and the bearing stator assembly 130. The motor side end wall 32 and bearing side end wall 34 of the rotor may be configured to have a generally flat, smooth surface presented toward the motor stator and bearing stator assemblies 120 and 130, respectively. The opposing surfaces of the motor stator assembly 120 and the motor side end wall 32 and the opposing surfaces of the bearing stator assembly 130 and the bearing side end wall 34 and bearing stator assembly 130, being large, flat, smooth, and closely spaced, may help promote the squeeze film damping of the rotor 12.

In the illustrated embodiment, including the six bearing stator coil 132 configuration of the bearing stator 130 illustrated in Fig. 4B, the axial bearing stator core 134 may be fabricated by winding a thin flat strip of material in a spiral to form a cylinder. The cylinder is then machined to form the six stator posts 136 around which the wire is wound to form the coils 132. The axial bearing target 70 may have a similar wound construction. The flat strips of material used to form the axial bearing stator core 134 and axial bearing target 70 can have a high magnetic permeability. For example, the flat strips may be formed of a ferrite, powder iron, or laminated silicon steel.

The adjacent coils 132, wound in opposite directions with the same wire, induce magnetic flux in opposite axial directions in their respective stator posts 136. The oppositely wound coils 132 reduce the mutual inductance between the three bearing stator poles 132a-c. This allows the bearing stator poles 132a-c to operate without mutually interfering with each other. This configuration helps reduce the likelihood that a change in current in one of the bearing stator poles 132a-c due to a change in the magnetic gap 310 (see Fig. 6A) over that particular pole will result in inducing a change in current and force in the other two bearing stator poles.

The axial bearing stator assembly 130 and the axial bearing target 70 are important elements in the design of the axial bearing 286. It may be desirable that the material used to construct the axial bearing stator core 134 have a high saturation flux density so that the axial force density (i.e., the force per unit of stator volume) capability of the stator assembly 130 is high. It is also desirable the magnetic permeability of the material used to construct the stator core 134 is high to help keep low the coil losses of the axial bearing 286. If the magnetic permeability of the stator core 134 is low, then additional amp-turns must be generated by the axial bearing coils 132 to induce the required level of magnetic flux density in the air gap between the stator 130 and the axial bearing target 70. Higher coil amp-turns require either more current through the same number of coil turns, or the same current though a larger number of coil turns. In either case, the coil $i^2R$ (power) loss associated with a given coil geometry is increased.

It may also be desirable that the axial bearing stator 130 be operated at a flux density sufficiently far removed from magnetic saturation to help avoid detrimental effects on axial bearing behavior that can result from permeability nonlinearities that typically accompany the approach to magnetic saturation. The typical rapid decrease in magnetic permeability results in a corresponding decrease in stator leg air gap flux density with an associated decrease in coil self-inductance. In a sense, the decrease in permeability as a material approaches saturation can have an effect similar to an increase in stator to target air gap. Consequently, the rapid drop in permeability as the stator material approaches magnetic saturation can significantly alter the shape of the force characteristic curve shown in Figure 6B, and can result in the bearing being inoperative.

Those skilled in the art will appreciate that the axial bearing stator core 134 can be made from a variety of magnetic materials, such as a ferrite material, a sintered iron material, or a high performance metallic lamination. Ferrite materials typically have a low saturation flux density (typically 0.4 to 0.5 Tesla), which can limit the flux density generated in the stator to target air gap and thereby can limit the force density of the axial bearing 130. Sintered iron material has a flux saturation flux density (typically 0.8 Tesla) greater than that of the ferrite material, but also has a low permeability. The lower permeability of the sintered iron material may require that a high coil current be used in order to take advantage of the higher flux density capability of the sintered iron material. This, however, can result in higher coil $i^2R$ losses. A laminated stator material may have both high flux density and high permeability. For example, a silicon steel or cobalt iron alloy material can be used to construct an axial bearing stator 130 with both high force density and low coil loss.

High performance metallic stator materials tend to be good electrical conductors and their use in electrical equipment operating at high frequencies such as those used in the axial bearing 286 may necessitate that the stator materials be laminated in order to reduce eddy current losses. In doing so, it may also be desirable to laminate the stator material in a manner consistent with low manufacturing cost. This can be achieved in various manners. For example, the stator materials may be laminated with tapered radial laminations, with laminations bent in a curve similar to a logarithmic spiral, or by spiral winding a thin strip of magnetic material about a cylindrical mandrel into a cylinder pre-machined stator precursor. Spiral winding may be more desirable than tapered laminations and bent curve laminations since the magnetic flux tends to be directed in the circumferential direction in the flux path of the target and in the regions of the stator below the excitation coils.

In an example configuration, the spiral winding approach was used for the fabrication of both the axial bearing stator core 134 and the axial bearing target 70. For the stator core 134, a thin 0.004" by 0.5" wide by 290" long strip of silicon iron was spiral wound about a 0.70" diameter cylindrical mandrel to form a stator precursor. After removal from the mandrel, the upper and lower faces of the resulting spiral wound stator precursor were machined flat. After this machining resulted in a stator cylinder of the correct height, coil slots defining the stator posts 136 were cut into the cylinder using a machining method, such as milling, grinding, or EDM machining. The latter method may be preferable since it tends to eliminate smearing of lamination material from lamination to lamination. This smearing can result in local short circuits between laminations which tend to defeat the purpose for using separate thin metallic laminations that are electrically insulated from one another.

The axial bearing target 70 was fabricated in a similar fashion. A thin, long strip of silicon iron spiral was wound around a cylindrical mandrel to form a target precursor. After removal from the mandrel, the upper and lower faces of the resulting spiral wound target precursor were machined flat to form the target 70. Since the target 70 is in the shape of a simple washer, no additional machining was required after machining its upper and lower surfaces flat and to the associated proper washer height.

Thus far, the pump 10 has been illustrated as having a dual radial bearing configuration including the motor side radial bearing 282 and the bearing side radial bearing 284. The pump 10 could, however, be configured to have a single radial bearing. In this instance, for example, the bearing side radial bearing 284 could be omitted and the motor side radial bearing 282 could be configured to provide the desired radial stiffness and axial pull to oppose the axial bearing 286. As another example, the motor side radial bearing 282 could be omitted and the bearing side radial bearing 284 could be configured to provide the desired radial stiffness and axial pull to oppose the axial bearing 286. The dual radial bearing configuration, however, can help provide added adjustability.

According to the present invention, the dual radial bearing configuration using the motor side radial bearing 282 and the bearing side radial bearing 284 allows for adjustment of the net axial force imposed on the rotor 12 by the radial bearings independently from the adjustment of the radial stiffness imposed on the rotor by the radial bearings. According to the present invention, adjustment of the motor side radial bearing 282 and bearing side radial bearing 284 is achieved by adjusting the position of the PM motor side radial bearing structure 250, the PM bearing side radial bearing structure 252, or both. These adjustments may be performed, for example, by adjusting the fastening means 270 and 272 that are used to connect the motor side radial bearing 250 and bearing side radial bearing 252 to the housing 14.

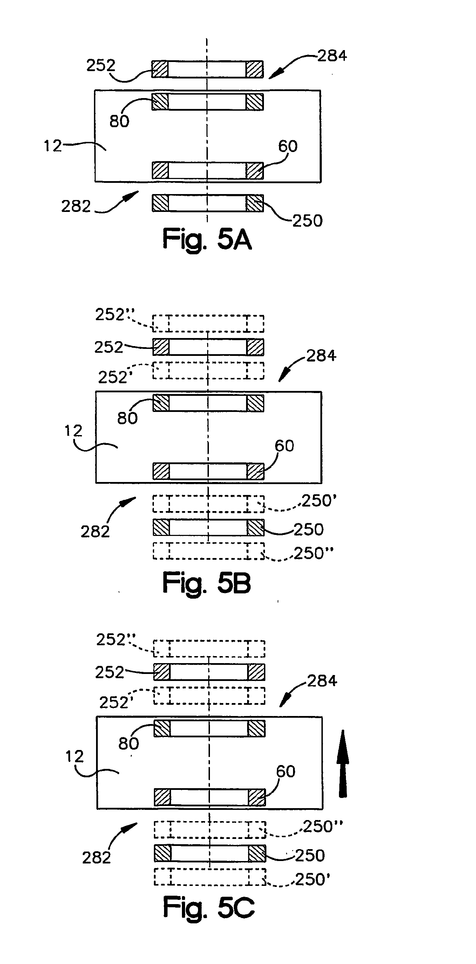

Adjustment of the motor side radial bearing 282 and the bearing side radial bearing 284 is shown in Figs. 5A-5C. Referring to Fig. 5A, the motor side radial bearing 282, i.e., the motor side PM radial bearing ring 60 and the PM motor side radial bearing structure 250, and the bearing side radial bearing 284, i.e., the bearing side PM radial bearing ring 80 and the PM bearing side radial bearing structure 252, are illustrated schematically.

To adjust the radial stiffness of the bearings 282 and 284 independently of the net axial pull exerted on the rotor 12 by the bearings, the motor side radial bearing 250 and stator side radial bearing 252 are moved either toward or away from each other in equal amounts. This is shown in Fig. 5B. Referring to Fig. 5B, to increase the radial stiffness of the bearings 282 and 284 without affecting the net axial pull exerted on the rotor 12, the motor side and stator side radial bearings 250 and 252 are moved equal distances toward each other. This is illustrated at 250' and 252' in Fig. 5B. To decrease the radial stiffness of the bearings 282 and 284 without affecting the net axial pull exerted on the rotor 12, the motor side and stator side radial bearings 250 and 252 bearings are moved equal distances away from each other. This is illustrated at 250" and 252" in Fig. 5B.

To adjust the net axial pull exerted on the rotor 12 by the bearings 282 and 284 independently of the radial stiffness with which the bearings support the rotor, the motor side radial bearing 250 and stator side radial bearing 252 are moved relative to the rotor with their position relative to each other remaining unchanged. Thus, in adjusting the net axial pull, the motor side and stator side radial bearings 250 and 252 are moved equal distances relative to the rotor 12, one bearing moving toward the rotor and the other bearing moving away from the rotor. This is shown in Fig. 5C. Referring to Fig. 5C, to increase the net axial pull of the bearings 282 and 284 in the direction of the arrow in Fig. 5C, the motor side and stator side radial bearings 250 and 252 are moved toward the positions illustrated at 250' and 250' in Fig. 5C. To decrease the net axial pull of the bearings 282 and 284 (i.e., increase the net axial pull in the direction opposite the arrow in Fig. 5C), the motor side and stator side radial bearings 250 and 252 are moved toward the positions illustrated at 250" and 252" in Fig. 5C. Because the axial force and radial stiffness are non-linear functions relative to the magnet gap, independent adjustment is possible when the adjustments are small relative to the total gap between magnets (e.g., <20%). As the adjustments get larger than this, second-order effects are more pronounced, and the adjustments become less independent.

As a further feature of the radial bearing design of the present invention, the motor side radial bearing 282 and the stator side radial bearing 284 may be offset from each other to help negate an average net hydraulic radial force exerted on the rotor 12. More specifically, the motor side PM radial bearing ring 60, PM motor side radial bearing structure 250, bearing side PM radial bearing ring 80, and the PM bearing side radial bearing structure 252 may be offset relative to each other to exert a radial force on the rotor 12 opposite the average net hydraulic radial force. This may help the rotor 12 to run more centered on the axis 16.

**First Example Pump Configuration**

In a first example configuration, a rotary pump is constructed in accordance with the present invention. The first example pump configuration is of the disposable configuration, omitting the bearing assembly seal and the motor assembly seal, as described above. For the first example pump configuration, each motor stator coil is made of eight layers of 25-gage copper wire, and occupies 0.16-in of space. The motor magnets and coils are positioned on silicon-steel cores that serve as flux return paths. The cores are thin, with the intent to choke the flux to help minimize flux changes with motor gap changes. This helps reduce the negative stiffness of the motor.

The flux density distribution was estimated using a two-dimensional finite element analysis program and the results indicate a calculated peak flux density of 0.25 tesla at the middle of the coil space. Using an axial gap motor analysis program and assuming this sinusoidal peak flux density of 0.25 tesla, the calculated motor efficiency is 70% for 5.3-W output at 2,700 rpm and 5 liter/min at 100 mm-Hg. Thus, the motor input power will be 7.6 W (5 ÷ 0.70). Allowing approximately 2 W for axial bearing operation, the total power consumption for this first example pump configuration is about 9.6 W. At the 4,300-rpm speed, the motor efficiency is about 70%, which equates to an overall power consumption of about 20.6 W.

For a nominal magnetic gap of 0.19-in. from rotor magnet surface to stator core (0.030-in. walls/fluid gap plus 0.160-in. coil thickness), the axial pull of the motor was calculated to be 1.94 lb ($F_{motor}$). Reducing the gap by 0.005-in. increases this force to 2.07 lb. Using these values, the axial stiffness is calculated to be -26.0 lb/in. ($K_{amotor}$), and the angular stiffness is thus:

$$K_{\theta motor} = K_{amotor}(R_o{}^2 + R_i{}^2)/4 = (-26.0)(.75^2 + .25^2)/4 = -4.06 \text{ lb-in./rad}$$

These values for motor stiffness are used in our estimation of rotor critical speeds, as shown below.

In the first example pump configuration, a single radial bearing design is employed and the pump includes only a bearing side radial bearing, as described above. The motor side PM radial bearing ring and the PM motor side radial bearing structure of the radial bearing are axial polarized (1.0-tesla remnant flux density) magnet rings with a 0.55-in. OD and a square cross section of 0.1-in. Using a PM bearing analysis program, the stiffness and axial pull versus gap values are calculated as shown in Table 1.

**Table 1. Radial Bearing Force and Stiffness**

| Gap, in. | $F_{calc}$, 1b | $K_{rcalc}$, 1b/in. | $K_{acalc}$, 1b/in. | $K_{\theta calc}$, 1b-in./rad |
|---|---|---|---|---|
| 0.03 | 2.06 | 22.3 | -44.5 | -2.25 |
| 0.04 | 1.67 | 16.8 | -33.6 | -1.7 |
| 0.05 | 1.38 | 13.0 | -26.0 | -1.32 |

In the first example pump configuration, the bearing stator assembly has the three bearing stator coil configuration, as described above (see Fig. 4B). The pole area per LCR circuit is approximately 0.190-in.$^2$, and the coil slot radial width is 0.120-in. 200 turns of 32-gage copper wire were used (0.008-in. copper diameter; 0.009-in. diameter with enamel insulating coating). The slot depth is about 0.240-in. The wire resistance is approximately 6.9 ohm (R). For the rotor to operate at 0.015-in. magnetic gap (walls plus fluid space), a tuning inductance of 4.66 x 10$^{-3}$ Henry (L) calculated at 0.013 in is selected. Using a capacitor of 2.5 x 10$^{-6}$ Farad (C), the calculated resonance frequency of the LCR circuit is 1474 Hz ($\omega_n$ = 9,257 rad/sec). The amplification factor of this resonance is approximately Q = $L\omega_n/R$ = 6.2.

The response peak is wide enough to accommodate 0.010-in. of rotor axial displacement. The rotor should be confined in the axial displacement range of ±0.005-in. from the operating gap at 0.015-in. The first example pump configuration allowed 0.010-in. of fluid flow space and 0.005-in. walls. In this range, the average axial stiffness was 26 1b/in. per LCR circuit, or a total of 78 lb/in.

The weight of the rotor 12 is estimated to be about 0.083 lb. At the preset gap of 0.013-in., the axial pull per LCR is calculated to be 0.08 lb for a total of 0.24 lb. The steady-state force balance requires:

$$F_{calc} = -3 F_{lcr} + F_{motor} - Mg = -0.24 + 1.94 - 0.083 = 1.617 \text{ lb}$$

This assumes that the motor is on top and the impeller weight acts against the motor pull. By interpolation from rows 2 and 3 of Table 1, the radial bearing gap will be 0.042-in., and the stiffness of the radial bearing is $K_{rcalc}$ = 16.1 1b/in., $K_{acalc}$ = -32.2 1b/in. and $K_{\theta calc}$ = -1.63 lb-in./rad. The sum of motor and radial bearing negative axial stiffness is 58.2 1b/in. (= 26.0 + 32.2), which is less than the total axial bearing positive axial stiffness (78 lb/in.). Similarly, the axial bearing provides more angular stiffness, i.e., 11.0 in.-lb/rad (= 1.5 x 26 x 0.531$^2$) than the total negative angular stiffness of the motor and radial bearing. This result ensures that there is sufficient force and axial and angular stiffness to levitate and support the rotor.

The analysis indicates a power loss of 0.67 W for each of the bearing LCR circuits, or 2.0 W total for the three bearing LCR circuits of the axial bearing stator. The corresponding coil peak current is 0.44 A, which corresponds to a coil slot current density of:

$$J = (0.44)(200)/(0.12)(0.24) = 3056 \text{ A/in}^2 = 4.7 \text{ A/mm}^2$$

This coil slot current density is an acceptable value with nominal amounts of fluid cooling.

As described above, the weight (M) of the rotor 12 will be 0.083 lb. The rotor inertias are:

$$\text{Polar moment of inertia: } I_p = MR^2/2 = (0.083)(0.75)^2/2 = 0.0233 \text{ lb-in.}^2$$

$$\text{Transverse moment of inertia: } I_t = M(R^2/4 + L^2/12) = 0.0128 \text{ lb-in.}^2;$$

for L=0.4-in.

Because, in the first example pump configuration, the radial bearing is at only one side of the rotor, the lateral and rocking modes have mixed mode shapes, i.e., mixing with both radial and rocking motions. Using the above mass inertial and stiffness data, we calculated the first (radial dominating) critical speed at 2,600 rpm and a very high second (rocking dominated) critical speed due to the gyroscopic effect. The axial critical speed is determined to be 2,900 rpm.

The critical speeds will be influenced by the effective mass added to the impeller as a result of its motion within the fluid being pumped. For a blood pump, the added masses for blood are:

$$\text{Radial mode: } M_{ra} = \rho \pi R^2 L = 0.027 \text{ lb}$$

$$\text{Axial mode: } M_{aa} = (8/3)\rho R^3 = 0.043 \text{ lb}$$

These values are significant portions of the estimated rotor mass of 0.083 lb. Their effect will be to lower the critical speeds to 2,258 rpm (radial) and 2,354 rpm (axial). These two calculated critical speeds are respectively 16% and 13% lower than the lower operating speed (2,700 rpm). The calculated critical speeds could be detuned to be acceptable by slightly reducing the stiffness. Damping in the rotor dynamic system may also help traverse the critical speed.

The first example pump configuration exhibited sufficient stiffness in the bearing system to resist imposed shock and vibration loads. Bearing power consumption was below 2.5 W at all operating conditions. The pump exhibited the ability to pump 5 liter/min at 100 mm-Hg pressure rise, with a motor input power below 8 W. The pump also exhibited the ability to pump 4 liter/min at 350 mm-Hg pressure rise, with motor power below 25 W. The pump further exhibited the ability to pump 9 liter/min, 120 mm-Hg pressure rise, with 40-W maximum power.

The first example pump configuration, described above and constructed in accordance with the present invention, provided high stiffness, good power efficiency, and high reliability without position sensors or active feedback electronics. The inclusion of the LCR axial bearing structure helps eliminate the need for a shaft seal and helps provide a clean wash flow patterns. In a blood pump implementation, this will help reduce the rate of thrombus formation in the pump. The magnetic axial bearing is also more durable than the ball bearings or bushings. The relatively small size of the pump in the first example configuration (6.4 cm diameter by 3.2 cm thick) may also be desirable.

**Second Example Pump Configuration**

In a second example configuration, a rotary pump is constructed in accordance with the present invention. The second example pump configuration is of the reusable configuration and thus includes the bearing assembly seal and the motor assembly seal, as described above. The motor stator assembly and axial bearing stator assembly of the second example pump configuration are reusable. For the second example pump configuration, the pump includes an axial bearing having the six coil configuration of Fig. 4A. Also, the rotor diameter is decreased to 1.5 inches in order to reduce viscous drag losses by more than the resulting speed increase, and in order to reduce the motor torque output required for a particular hydraulic output.

The 6-pole stator core for the axial bearing of the second example pump configuration is made of laminated steel. A flat strip or strips of laminated silicon-steel are wound to form an axial bearing target that is fixed to the rotor. As an example, a strip of laminated silicon-steel that is 0.1-mm thick and 3.0-mm wide may be wound to form a 3.0-mm thick axial bearing target on the rotor. Thin-film barriers are used to form the motor seal and bearing seal assemblies that seal the motor stator and axial bearing stator from the rotor. On the motor side, the volute design from the implantable pump is modified to accommodate the annular bead in the form of a machined polycarbonate ring. A 20-mil-thick (0.25 mm) sheet of polycarbonate forming the membrane portion is glued to the ring and the inlet port is glued to the polycarbonate sheet. On the bearing side, a 2-mil-thick (0.05 mm) sheet of adhesive-backed polyester material forming the membrane portions is mounted on a metal ring forming the annular bead and the assembly is secured in the stator housing with an O-ring to seal the axial bearing stator from the rotor.

In the second example configuration of the pump, the axial-gap motor has a measured power capacity of 27 W and a torque constant of approximately 0.01 Nm/A. The entire motor core, including the posts, is made of powdered iron so as to reduce eddy current loss. The iron posts form a part of the flux return path formed by the core of the motor. Without the posts, the magnetic flux would flow evenly between the magnets and the core. With the posts, the flux tends to concentrate and pass through the bearing coils. In this way, the posts serve to increase the magnetic flux link of the coils and thus produce more torque per unit of coil current. Since the motor PM ring on the rotor has eight poles, the magnetic flux in the core fluctuates at a frequency of 500 Hz or higher.

The poles on the core may also increase the negative axial and angular stiffness on the rotor. The negative axial stiffness can be compensated for by the use of higher coil currents. Maintenance of the angular stiffness requires more "moment arm," or radial distance between the bearing poles and bearing center, because the positive angular stiffness is proportional to the square of the distance. Therefore, to compensate for the angular stiffness by increasing the moment arm, the bearing core pole areas in the ICB were moved out radially.

Laminated silicon steel is used to form the axial bearing stator and the axial bearing PM ring on the rotor. In the second example pump configuration, the dual radial bearing design (see Figs. 5A-5C) is employed. The second example pump configuration thus includes both a motor side radial bearing and a bearing side radial bearing.

During testing, the second example pump configuration was run safely at a maximum power of approximately 40 W without overheating the motor coils. Loss data was calculated from observed peak-to-peak values of the bearing sinusoidal currents, and the coil resistance of 3.8 ohms per LCR bearing circuit. The maximum recorded bearing power loss is identified as 1.7 W. Considering the eddy current and hysteresis loss of laminated-silicon-steel bearing cores, it was observed that, at approximately 900 Hz (the LCR bearing circuit excitation frequency) and an average estimated flux density of 4 kilogauss in the cores, the loss is approximately 1.0 W/1b of core material. Since the second example pump configuration includes about 0.12 lb of laminated cores, an additional loss of 0.12 W was estimated. Therefore, the total bearing loss for the second example pump configuration for all operating conditions is calculated at no more than 1.82 W (1.7+0.12).

With shocks imposed at 32-g in the axial direction and 42-g in the vertical direction loads, the LCR bearing circuit currents showed insignificant response. The second example pump configuration thus demonstrates sufficient stiffness in the radial and axial bearings. The pump also exhibited the ability to pump 5 liter/min at 100 mm Hg pressure rise, with motor power input below 8 W; the ability to pump 4 liter/min at 350 mm Hg pressure rise, with motor power below 25 W; and the ability to pump 9 liter/min, 120 mm Hg pressure rise, with 40-W maximum power.

From the above description of the invention, those skilled in the art will perceive improvements, changes and modifications. Such improvements, changes and modifications within the skill of the art are intended to be covered by the appended claims.

**Claims**

1.  A pump comprising:

    a housing having a fluid inlet and a fluid outlet;

a rotor disposed within the housing and rotatable about an axis to move fluid from the fluid inlet to the fluid outlet; and

a magnetic axial bearing for supporting the rotor, the axial bearing comprising an axial bearing target disposed on the rotor and an axial bearing stator disposed on the housing, the axial bearing stator including multiple stator poles, each of the stator poles including a first coil portion wound in a first direction and a second coil portion wound in a second direction opposite the first direction.

2. The pump recited in claim 1, wherein the first direction is clockwise and second direction is counterclockwise.

3. The pump recited in claim 1, wherein each of the multiple stator poles comprises an electromagnet having an inductance (L), the electromagnet being connected in series with a resistance (R), a capacitance (C), and a phase voltage source to form an LCR circuit for controlling the axial bearing.

4. The pump recited in claim 1, wherein the bearing stator includes three stator poles, each stator pole including a first coil wound in a clockwise configuration and a the second coil wound in a counterclockwise configuration, each of the stator poles being excited by a corresponding electrical phase of a three phase voltage source.

5. The pump recited in claim 1, wherein the axial bearing stator comprises a strip of material wound in a cylindrical coil and machined to form a stator core including stator posts around which the first and second coils of each stator pole are wound.

6. The pump recited in claim 1, further comprising:

a magnetic first radial bearing for exerting a force on the rotor in a first direction along the axis; and

a magnetic second radial bearing for exerting a force on the rotor in a second direction along the axis opposite the first direction;

the first and second radial bearings being adjustable to allow for adjusting the net axial force exerted on the rotor by the radial bearings independent of the radial stiffness of the radial bearings.

7. The pump recited in claim 1, wherein the axial bearing stator is excited by voltage superimposed on a voltage supplied to a motor stator of the pump.

**Patentansprüche**

1. Pumpe, umfassend:

ein Gehäuse mit einem Fluideinlaß und einem Fluidauslaß;

einen Rotor, der innerhalb des Gehäuses angeordnet und um eine Achse drehbar ist, um Fluid aus dem Fluideinlaß in den Fluidauslaß zu bewegen; und

ein magnetisches Axiallager zum Lagern des Rotors, wobei das Axiallager eine Axiallageranode, die am Rotor angeordnet ist, und einen Axiallagerstator, der am Gehäuse angeordnet ist, umfaßt, wobei der Axiallagerstator mehrere Statorpole aufweist, wobei jeder der Statorpole einen ersten Spulenabschnitt, der in einer ersten Richtung gewickelt ist, und einen zweiten Spulenabschnitt, der in einer zweiten Richtung entgegen der ersten Richtung gewickelt ist, aufweist.

2. Pumpe nach Anspruch 1, wobei die erste Richtung der Uhrzeigersinn ist und die zweite Richtung der Gegenuhrzeigersinn ist.

3. Pumpe nach Anspruch 1, wobei jeder der mehreren Statorpole einen Elektromagneten mit einer Induktivität (L) umfaßt, wobei der Elektromagnet mit einem Widerstand (R), einer Kapazität (C) und einer Phasenspannungsquelle in Reihe geschaltet ist, um eine LCR-Schaltung zur Steuerung des Axiallagers zu steuern.

4. Pumpe nach Anspruch 1, wobei der Lagerstator drei Statorpole aufweist, wobei jeder Statorpol eine erste Spule, die in einer Uhrzeigersinn-Konfiguration gewickelt ist, und eine zweite Spule, die in einer Gegenuhrzeigersinn-Konfiguration gewickelt ist, aufweist, wobei jeder der Statorpole durch eine entsprechende elektrische Phase der Dreiphasenspannungsquelle erregt wird.

**5.** Pumpe nach Anspruch 1, wobei der Axiallagerstator einen Materialstreifen umfaßt, der in einer Zylinderspule gewickelt und bearbeitet ist, um einen Statorkern zu bilden, der Statorpfosten aufweist, um die erste und zweite Spulen jedes Statorpfostens gewickelt sind.

**6.** Pumpe nach Anspruch 1, ferner umfassend:

ein erstes magnetisches Radiallager zur Ausübung einer Kraft auf den Rotor in einer ersten Richtung entlang der Achse; und
ein zweites magnetisches Radiallager zur Ausübung einer Kraft auf den Rotor in einer zweiten Richtung entlang der Achse entgegen der ersten Richtung;

wobei das erste und das zweite Radiallager regulierbar sind, um eine Regulierung der Nettoaxialkraft zu ermöglichen, die durch die Radiallager auf den Rotor unabhängig von der Radialsteifigkeit der Radiallager ausgeübt wird.

**7.** Pumpe nach Anspruch 1, wobei der Axiallagerstator durch Spannung erregt wird, mit der eine Spannung überlagert ist, die einem Motorstator der Pumpe zugeführt wird.

**Revendications**

**1.** Pompe comprenant:

un logement ayant un orifice d'entrée de fluide et un orifice de sortie de fluide ;
un rotor disposé dans le logement et rotatif autour d'un axe pour déplacer le fluide de l'orifice d'entrée de fluide vers orifice de sortie de fluide ; et
un support axial magnétique pour supporter le rotor, le support axial comprenant une cible de support axial disposée sur le rotor et un stator de support axial disposé sur le logement, le stator de support axial comprenant de multiples pôles de stator, chacun des pôles de stator comprenant une première partie de bobine enroulée dans une première direction et une seconde partie de bobine enroulée dans une seconde direction opposée à la première direction.

**2.** Pompe selon la revendication 1, dans laquelle la première direction se fait dans le sens des aiguilles d'une montre et la seconde direction se fait dans le sens inverse des aiguilles d'une montre.

**3.** Pompe selon la revendication 1, dans laquelle chacun des multiples pôles de stator comprend un électroaimant ayant une inductance (L), l'électroaimant étant connecté en série avec une résistance (R), une capacité (C) et une source de tension de phase pour former un circuit LCR pour commander le support axial.

**4.** Pompe selon la revendication 1, dans laquelle le stator de support comprend trois pôles de stator, chaque pôle de stator comprenant une première bobine enroulée selon une configuration dans les sens des aiguilles d'une montre et une seconde bobine enroulée selon une configuration dans le sens inverse des aiguilles d'une montre, chacun des pôles de stator étant excité par une phase électrique correspondante d'une source de tension triphasée.

**5.** Pompe selon la revendication 1, dans laquelle le stator de support axial comprend une bande de matériau enroulée dans une bobine cylindrique et usinée pour former un noyau de stator comprenant des montants de stator autour desquels les première et seconde bobines de chaque pôle de stator sont enroulées.

**6.** Pompe selon la revendication 1, comprenant en outre :

un premier support radial magnétique pour exercer une force sur le rotor dans une première direction le long de l'axe; et
un second support radial magnétique pour exercer une force sur le rotor dans une seconde direction le long de l'axe opposé à la première direction;
les premier et second supports radiaux étant ajustables pour permettre l'ajustement de la force axiale nette exercée sur le rotor par les supports radiaux indépendamment de la rigidité radiale des supports radiaux.

**7.** Pompe selon la revendication 1, dans laquelle le stator de support axial est excité par une tension superposée sur une tension fournie à un stator de moteur de la pompe.

Fig. 1

Fig. 2

Fig. 3A

Fig. 3B

Fig. 4A

Fig. 4B

252 284
80
12
60
282 250

**Fig. 5A**

252''
252
252'
284
80
12
60
250'
282 250
250''

**Fig. 5B**

252''
252
252'
284
80
12
60
250''
282 250
250'

**Fig. 5C**

**Fig. 6A**

STEADY-STATE
FORCE

OPERATING GAP    MAXIMUM GAP

**Fig. 6B**

**EP 1 668 764 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 50861803 P
- US 5078741 A